# EUROPEAN PATENT APPLICATION

(11) **EP 4 279 017 A1**
(43) Date of publication of application: **22.11.2023**
(21) Application number: 23174219.8
(22) Date of filing: 19.05.2023
(51) Int. Cl.: A61B 90/50, A61F 2/08

(54) **TISSUE GRAFT PREPARATION SYSTEM**

(30) Priority: 20.05.2022 US 202263344401 P; 08.07.2022 US 202263359320 P
(71) Applicant: Arthrex Inc, Naples, FL 34108-1945 (US)
(72) Inventor: XEROGEANES, John, Naples, 34108 (US); ROQUE, Samuel, Naples, 34108 (US); INGWER, Zachary, Naples, 34108 (US); JOLLY, Jake, Naples, 34108 (US); BOYLE, Justin, Naples, 34108 (US)
(74) Representative: Lohr, Jöstingmeier & Partner

(57) **Abstract**

A support system is disclosed to prepare harvested soft tissue for grafting. Methods of graft preparation are also disclosed.

## Description

### BACKGROUND

Completely replacing damaged tissue often requires using harvested tissue, such as tendons or ligaments, to implant into a patient. The harvested tissue can be tensioned during its preparation for implantation. The harvested tissue is typically prepared by whip stitching various aspects of the harvested tissue, such as the ends, to prepare it for use. When using conventional graft preparation boards, a harvested tissue is attached to clamps on the board and pulled tight. Medical personnel then stitch desired portions of the harvested tissue with a suture. When suturing the tissue on the board, the person must pass the suture under the tissue and switch hands with the suture after each stitch in preparation for the next stitch. Doing so typically causes the loss of tension in the suture resulting in undesirable slack being introduced to the suture.

### SUMMARY

A graft support system configured to support a piece of tissue, such as, but not limited to, a graft, in a manner to enable graft preparation is disclosed. In particular, the graft support system may be configured to enable sutures to be easily passed around a piece of tissue held securely above a surface while the suture remains under tension to better facilitate graft preparation with whip stitching and the like. The graft support system may include a suture receiver configured to receive a suture under tension in a suture receiving opening and release the suture under tension from a suture release opening after moving a suture receiver base from a first position in which the suture receiving opening is open to a second position in which the suture release opening is open. As such, the suture is moved from one side of the tissue securement assembly to the other side easily, quickly and without losing tension on the suture.

In at least one embodiment, the graft support system may be configured to support one or more tissue securement assemblies, such as, but not limited to one or more posts or tissue clamps arms forming a tissue clamp, above a surface, such as, but not limited to, a system base. The tissue receiver may be supported by a movable suture receiver, which is in turn supported by a suture receiver support. In at least one embodiment, the suture receiver support may be a shaft, which may be, but is not required to be, removable from a system base. The movable suture receiver enables a suture to be held under tension and moved from one side of the graft support system to another side.

The graft support system may include a tissue receiver support base configured to support one or more tissue receivers, such as but limited to being, a tissue receiver, which may be, but is not limited to being, one or more posts or tissue clamps. The tissue receiver support base may be coupled to the suture receiver. The suture receiver may in turn be supported by a suture receiver support. As such, the graft support system may include a suture receiver supported remotely from the surface by the suture receiver support and may be configured to support the tissue receiver support base remote relative to the surface by the suture receiver support. The suture receiver may be configured to enable a stitch to be formed in the tissue, and the suture to be looped around the tissue while maintaining the suture under constant tension by receiving a portion of a suture under tension extending from a tissue held within the tissue receiver. As such, the portion of the suture under tension remains under tension by force applied to the suture such that the portion of the suture under tension is manipulated from a first side of the suture receiver to a second side of the suture receiver. In such configuration, a user may be better able to perform desired tissue preparation with whip stitching and the like during tissue preparation, such as, but not limited to, graft preparation by keeping the suture under constant tension.

In at least one embodiment, the suture receiver may be formed from a suture receiver base configured to slidably receive a suture receiver body. In such configuration, the suture receiver base may be slidable relative to the suture receiver body or vice versa. The suture receiver body may include a base support member and first and second arms separated to form a suture receiving chamber. In a first position, a suture receiving opening is open on a first side of the suture receiver. At the same time, the suture release opening on the second side is closed and the second arm, which is on the second side is supporting the suture receiver. The first side is unsupported and receives a suture under tension from the first side of the suture receiver via the suture receiving opening. The suture receiver base of the suture receiver may be moved to a second position in which the suture release opening is open and the suture receiving opening is closed. In this position, the suture receiver is supported by the first side and releases a suture under tension from the second side of the suture receiver via the suture release opening.

A method of preparing a graft for implantation or other use may include graft support system being used to grasp and hold a piece of tissue under tension above a surface and may entail the first and second tissue securement assemblies securely grasping a piece of tissue to support the tissue above the support base such that suture attached to the tissue remains under tension while being passed from one side to another side without having to pass the suture from one hand to another and possibly lose tension. The method may include a piece of tissue being held securely by the second tissue securement assembly. The piece of tissue may be pulled tight and moved between the distal ends of the tissue receiver, such as, but not limited to, a post or a tissue clamps formed from first and second clamp arms so that the graft punch may grasp the piece of tissue and retain the tissue extending between the first and second tissue securement assemblies. With the first and second clamp arms in an open position in which the graft punch is separated from the orifice, such as when the first and second clamp arms are separated to a maximum open position as limited by stop, the piece of tissue may be properly positioned for engagement and the second clamp arm may be pushed towards the first clamp arm via the thumb pad or by pushing the second clamp arm towards the first clamp arm. When using a tissue receiver formed from a post, the tissue material, such as a graft, may be attached to the post by inserting the post into a hole in the tissue material.

The method of preparing a graft may include, once the piece of tissue is secured and extending between the first and second tissue securement assemblies, passing a needle through the tissue and pulling the suture tight to place the suture under tension. The method may include pulling the tag end of the suture tight to place the suture under tension. The portion of the suture under tension may be pulled into the suture receiving chamber via the suture receiving opening of the suture receiver on the first side of the suture receiver. In such method, the portion of the suture under tension may remain under constant tension. The method may include moving the suture receiver body from the first position to the second position whereby the suture release opening is opened and the suture receiving opening is closed. The method may include moving the suture under tension within the suture receiving chamber and out of the suture release opening. Doing so enables the suture under tension to be moved from the first side of the tissue securement assembly to the second side. In other words, a user may pull the suture tight and move the suture from the first side of the tissue securement assembly to the second side while keeping the suture under tension. The tensioned tag end of the suture is then ready for the next stitch. The method may be replicated numerous times until a piece of tissue has been fully prepared.

A method of preparing a graft, may comprise: supporting a piece of tissue via a graft support system such that the piece of tissue is supported away from at least one surface; placing at least a portion of a tag end of a suture under tension such that the suture attached to the piece of tissue is under tension; and passing the tag end of the suture from a first side of the graft support system to a second side of the graft support system while retaining the suture and at least a portion of a tag end under tension such that the tag end of the suture is in position to create another stitch within the piece of tissue.

The method may further comprise: passing the tag end of the suture from a first side of the graft support system to a second side of the graft support system while retaining the suture and at least a portion of a tag end under tension comprises passing the tag end of the suture from a first side of the graft support system to a second side of the graft support system without a user having to relinquish grip of the tag end.

The method may further comprise: supporting a piece of tissue via a graft support system such that the piece of tissue is supported away from a surface comprises supporting a piece of tissue via a graft support system comprising a suture receiver supported remotely from the at least one surface and configured to support at least one tissue receiver remote relative to the at least one surface such that material can be passed between the at least one surface and the piece of tissue supported by the graft support system, and wherein
the method of preparing a graft may further comprise: wherein passing the tag end of the suture from a first side of the graft support system to a second side of the graft support system while retaining the suture and at least a portion of a tag end of the suture under tension comprises moving a portion of a tag end of the suture under tension on the first side of the graft support system into a suture receiving chamber of a suture receiver body of the suture receiver, moving a suture receiver body from a first position to a second position whereby a suture release opening is opened and a suture receiving opening is closed, and removing the tag end under tension from the suture release opening, thereby placing the tag end under tension on the second side of the graft support system.

An advantage of the graft support system is that the system enables a user, such as a medical professional, to keep tension on a suture in preparation for another stitch when moving a tensioned tag end of the suture from one side of a tissue securement assembly secured to a base to a second side of the tissue securement assembly without having to pass the suture from one hand to another. Rather, the tensioned tag end of the suture can remain in the same hand of the user.

Another advantage of the graft support system is that the system enables a user to rotate a tissue receiver so that a bottom surface of a tissue, such as a graft, is now a top surface for easy of inspection, for additional sutures or other work on the tissue. The tissue receiver may be rotated as desired and only limited by the tissue held within the tissue receiver.

Yet another advantage of the graft support system is that the suture receiver body is easily slidable from a first position to a second position to enable a user to easily move between first and second positions to enable a user to pass a suture from one side of a tissue securement assembly to another.

Another advantage of the graft support system is that the system is not limited to only using a tissue clamp as a tissue receiver but may also use one or more posts or other structure capable of securing at least a portion of a tissue.

Still another advantage of the graft support system is that the system, in embodiments in which the tissue receiver is a clamp, may include a clamp arm retainer configured to releasably secure the second clamp arm in a fixed position relative to the first clamp arm, whereby the first and second clamp arms releasably secure at least a portion of a tissue.

Another advantage of the graft support system is that the clamp arm retainer is easily actuated and controlled with a single finger of a user.

Yet another advantage of the graft support system is use of a suture card receiver slot on an arm forming a portion of a clamp to enable sutures to be held in place on the implant card while one or more sutures extending from the implant card is attached to a piece of tissue material.

Another advantage of the graft support system is that one or more support members configured to support the tissue receiver is removably attached to a surface, such as, but not limited to a base via a releasable connection system.

These and other features of the systems and methods of the graft support system are described in the following detailed description, drawings, and appended claims.

### BRIEF DESCRIPTION OF THE DRAWINGS

Figure 1 is a perspective view of the graft support system with a piece of tissue, which shown in this Figure 1 is a graft, extending between first and second tissue securement assemblies.
Figure 2 is a perspective view of the graft support system with a piece of tissue, which shown in this Figure 1 is a graft, extending between first and second tissue securement assemblies, whereby the first and second tissue securement assemblies each include suture receivers enabling one or more sutures to be easily passed around a piece of tissue held securely above a surface while the suture remains under tension.
Figure 3 is a perspective view of a first tissue securement assembly of the graft support system.
Figure 4 is a perspective view of the first tissue securement assembly of the graft support system with first and second clamp arms in an open position.
Figure 5 is an exploded perspective view of the first tissue securement assembly of the graft support system.
Figure 6 is an exploded perspective view with a different perspective than Figure 5 of the first tissue securement assembly of the graft support system.
Figure 7 is a perspective view of the first tissue securement assembly of the graft support system with first and second clamp arms in an open position whereby the first tissue securement assembly is supported by a system base.
Figure 8 is a detail view of a release connection system positioned distally of the suture receiver at a distal end of a support member.
Figure 9 is a top detail view of the suture receiver taken at detail 9-9 in Figures 3 and 12 and top view of Figure 13 with the suture receiver base in a first position with the suture receiving opening in an open position.
Figure 10 is a top detail view of the suture receiver taken at detail 9-9 in Figures 3 and 12 and top view of Figure 13 with the suture receiver base moving from the first position with the suture receiving opening in an open position, whereby the receiver is configured to receive a suture under constant tension on a first side of the receiver, to a second position with the suture release opening in an open position, whereby the receiver is capable of releasing the suture under constant tension on a second side of the receiver.
Figure 11 is a top detail view of the suture receiver taken at detail 11-11 in Figure 14 with the suture receiver base in a second position with the suture release opening in an open position.
Figure 12 is a perspective view of a first tissue securement assembly of the graft support system with the suture receiver base in a first position with the suture receiving opening in an open position.
Figure 13 is a detail view of the suture receiver with a suture receiver body restraint, which may be, but is not limited to being, a pin, positioned in the suture receiving chamber and attached to the suture receiver base to limit lateral movement of the suture receiver body when the suture receiver body contacts the suture receiver body restraint and movement stops.
Figure 14 is a perspective view of a first tissue securement assembly of the graft support system with the suture receiver base in a second position with the suture release opening in an open position.
Figure 15 is a top detail view of the suture receiver taken at detail 9-9 in Figures 3 and 12 and top view of Figure 13 with the suture receiver base in a first position with the suture receiving opening in an open position.
Figure 16 is a top detail view of the suture receiver taken at detail 11-11 in Figure 14 with the suture receiver base in a second position with the suture release opening in an open position.
Figure 17 is a perspective view of the first tissue securement assembly of the graft support system with an alternative suture receiver body restraint limiting movement of the suture receiver base between the first and second positions.
Figure 18 is a perspective view of an embodiment in which both first and second tissue securement assemblies include a suture receiver configured to receive a suture under tension in a suture receiving opening and release the suture under tension from a suture release opening after moving a suture receiver base from a first position to a second position.
Figure 19 is a perspective view of a rotatable support structure configured to enable the first and second tissue securement assemblies to be rotated along an axis generally aligned with a longitudinal axis of the tissue material extending between the first and second tissue securement assemblies.
Figure 20 is a perspective view of another embodiment of a tissue receiver.
Figure 21 is a perspective view of a tissue graft having holes configured to receive the tissue connection device, which may be a post, shown in Figure 20.
Figure 22 is a perspective view of the first tissue securement assembly of the graft support system with the suture receiver supported directly by a tissue receiver support base.
Figure 23 is a perspective view of the graft support system with a piece of tissue, which is a graft, extending between first and second tissue securement assemblies.
Figure 24 is a perspective view of the graft support system with a piece of tissue, which is a graft, extending between first and second tissue securement assemblies, as shown in Figure 6, whereby a needle has been inserted through the graft and a suture under tension has been inserted through the suture receiving opening into the suture receiving chamber when the suture receiver base is in the first position.
Figure 25 is a perspective view of the graft support system with a piece of tissue, which is a graft, extending between first and second tissue securement assemblies, as shown in Figure 6, whereby the suture under tension positioned in the suture receiving chamber is moved in the suture receiving chamber from near a first side of the suture receiver to a second side of the suture receiver, and the suture receiver base is moved from the first position to the second position.
Figure 26 is a perspective view of the graft support system with a piece of tissue, which is a graft, extending between first and second tissue securement assemblies, as shown in Figure 6, whereby the suture under tension is released from the suture receiving chamber by moving it through the suture release opening.
Figure 27 is a perspective view of the graft support system with a piece of tissue, which is a graft, extending between first and second tissue securement assemblies, as shown in Figure 6, whereby the suture under tension extends to the second side of the suture receiver after having been passed through the suture receiver.
Figure 28 is a perspective view of the graft support system with a piece of tissue, which is a graft, extending between first and second tissue securement assemblies, as shown in Figure 6, whereby the suture under tension is in a ready position for another stitch after having been passed through the suture receiver.
Figure 29 is a perspective view of the graft support system with a piece of tissue, which is a graft, extending between first and second tissue securement assemblies, as shown in Figure 6, whereby preparation on the graft has been finalized.

### DETAILED DESCRIPTION OF THE DRAWINGS

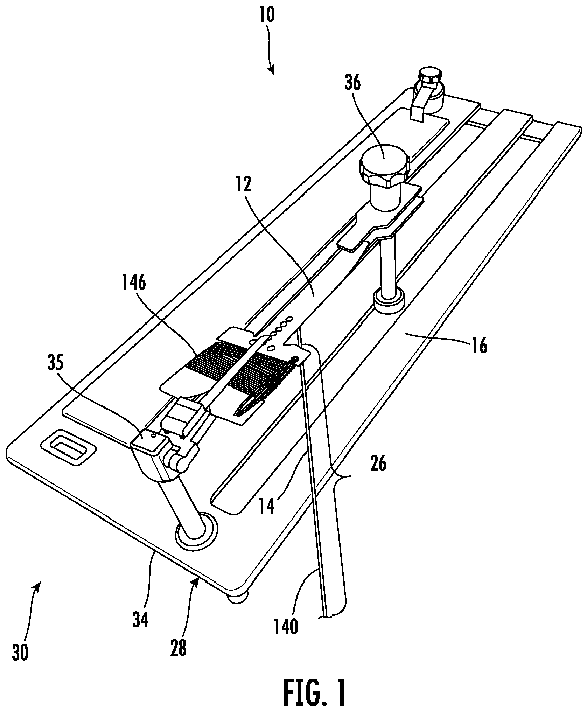

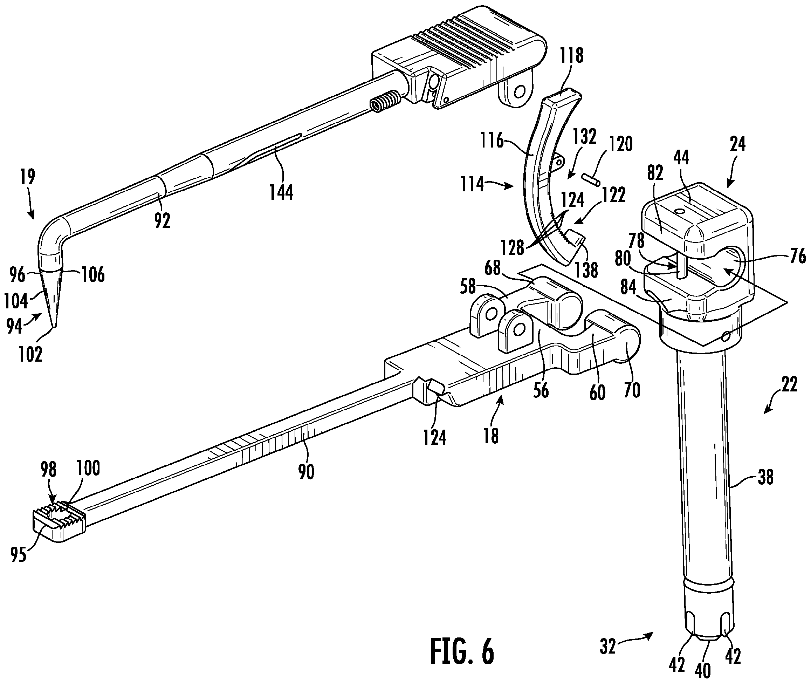

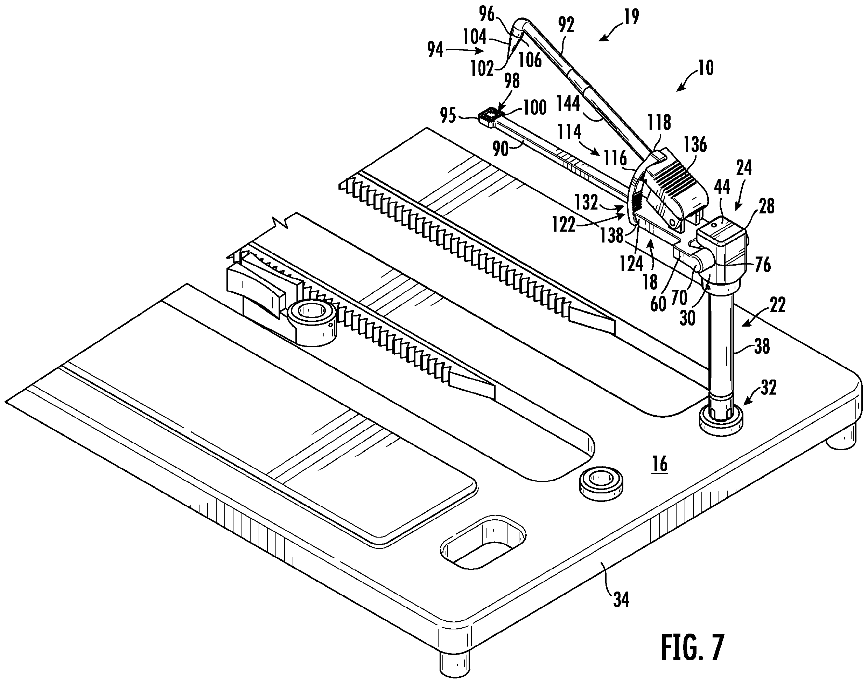

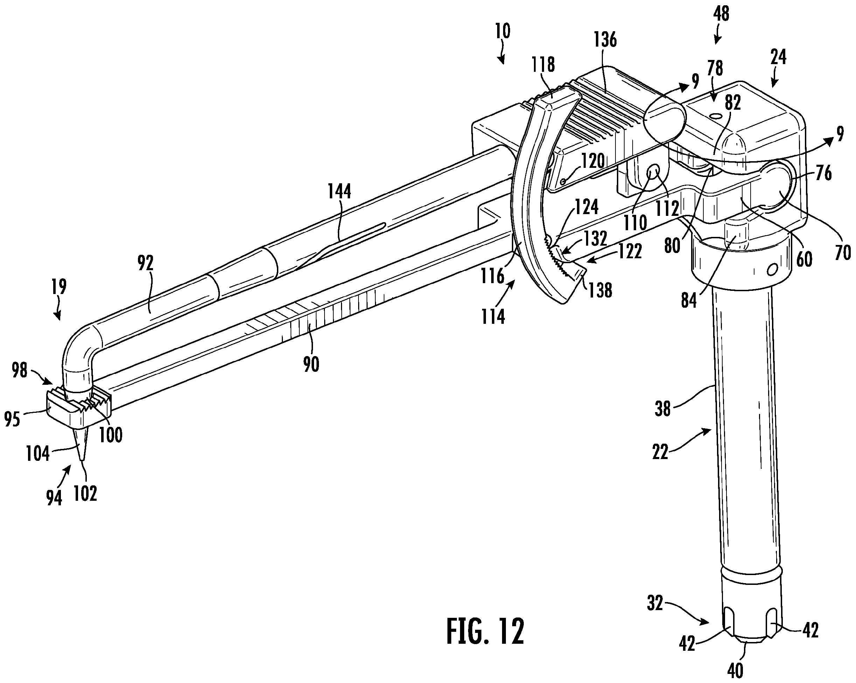

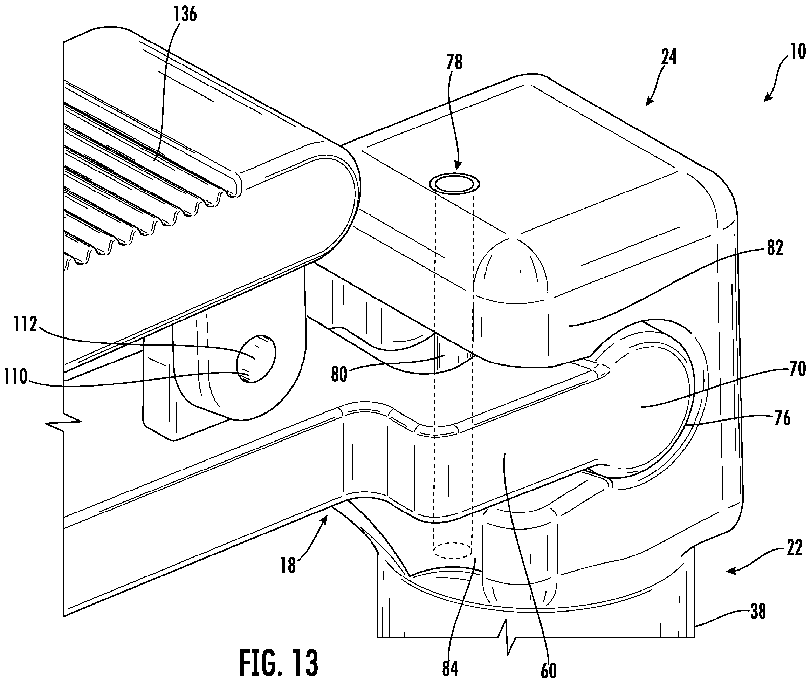

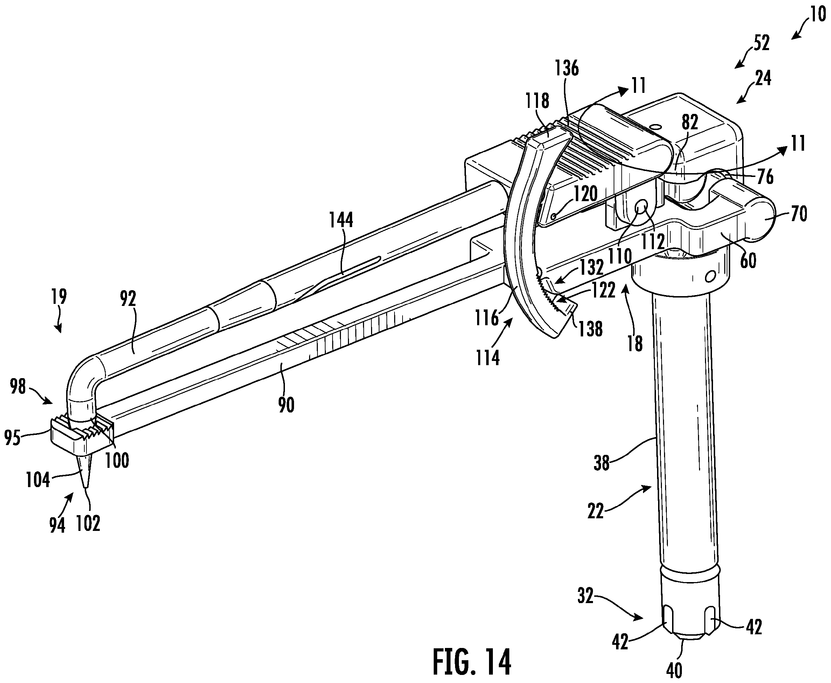

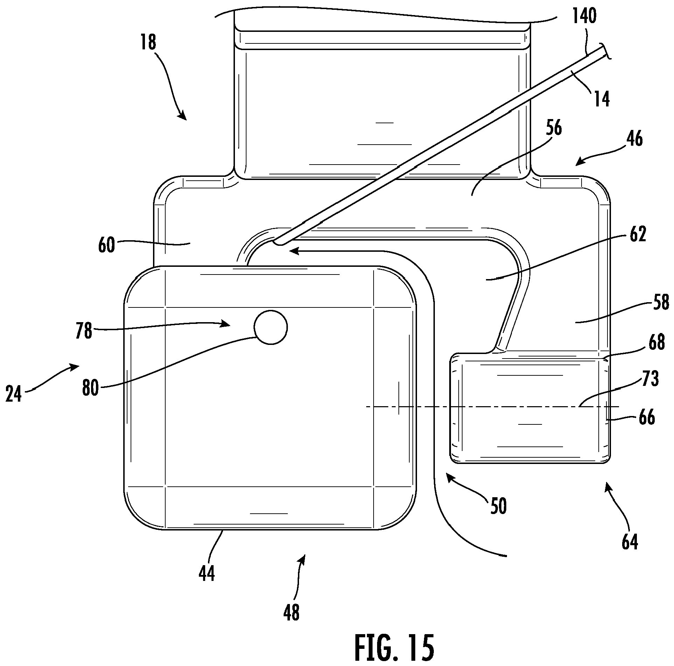

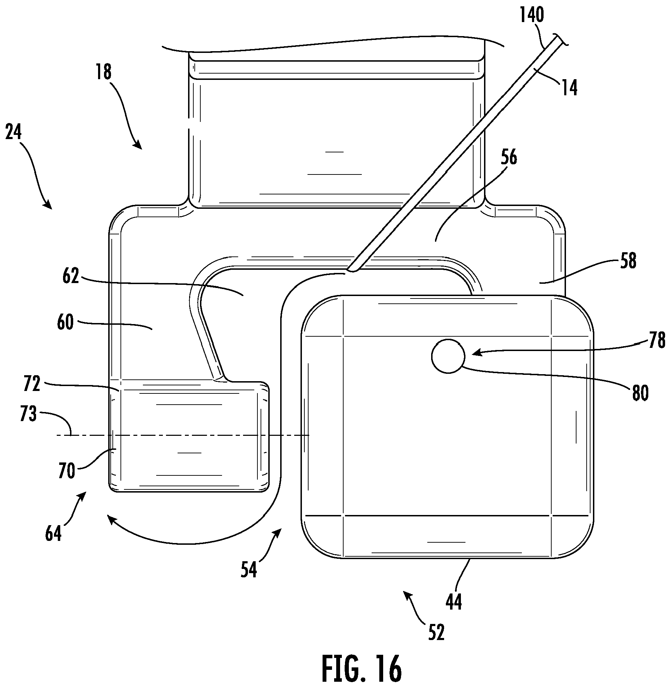

As shown in Figures 1-29, a graft support system 10 may be configured to support a piece of tissue 12, such as, but not limited to, a graft, in a manner to enable graft preparation. In particular, the graft support system 10 may be configured to enable sutures 14 to be easily passed around a piece of tissue 12 held securely above a surface 16 while the suture 14 remains under tension to better facilitate graft preparation with whip stitching and the like. The graft support system may include a suture receiver 24 configured to receive a suture 14 under tension in a suture receiving opening 50 and release the suture 14 under tension from a suture release opening 54 after moving a suture receiver base 44 from a first position 82 to a second position 84. As such, the suture 14 is moved from one side 28 of the tissue securement assembly 35 and suture receiver 24 to the other side 30 easily, quickly and without losing tension on the suture 14.

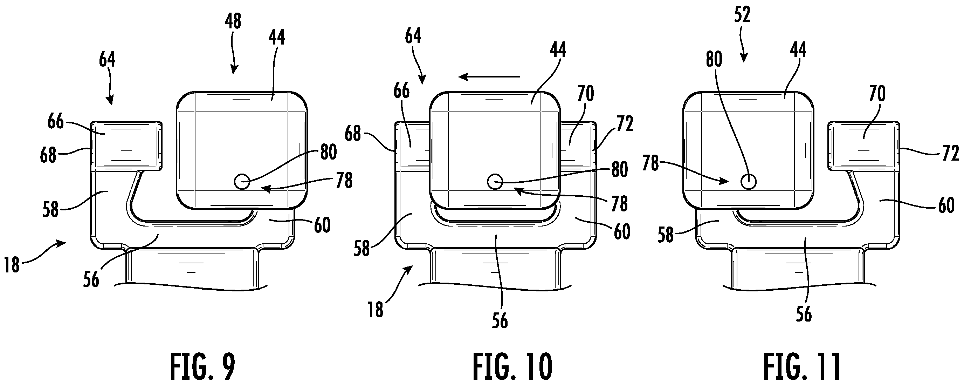

In at least one embodiment, the suture receiver 24 may be configured to move a suture receiver base 44 between a first position 48, as shown in Figure 9, thru a middle position, as shown in Figure 10, to a second position 52, as shown in Figure 11. The suture receiver 24 may receive a suture 14 under tension on a first side 28 of the suture receiver 24 when the suture receiver base 44 is in the first position 48 and release the suture 14 under tension on a second side 30 of the suture receiver 24 once the suture receiver base 44 has been moved to the second position 52. All the while, in at least one embodiment, the suture 14 has remained under constant tension. A user has not felt it necessary to switch hands with the suture 14 or in some manner lose tension on the suture 14.

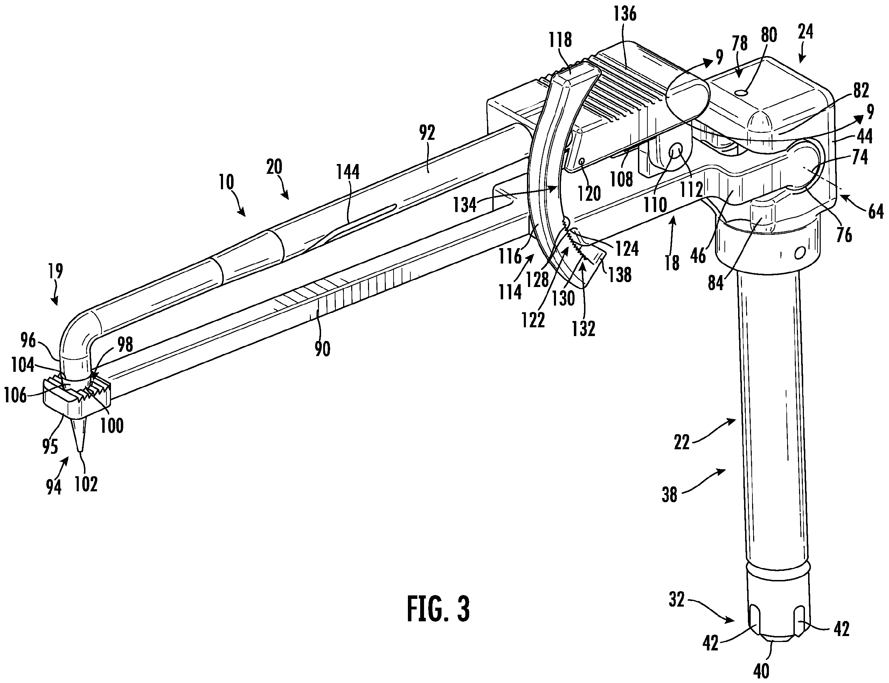

The graft support system 10 may include a tissue receiver support base 18, as shown in Figures 3-7, 12 and 17 configured to support one or more tissue receivers 19, which may be, but are not limited to being, a tissue clamp 20, and a suture receiver support 22 configured to support the tissue receiver support base 18 remote from the surface 16, which may be, but is not limited to being, a system base 34. The tissue receiver support base 18 may be coupled to the suture receiver 24. The suture receiver 24 may, in turn, be supported by a suture receiver support 22. In such a configuration, the suture receiver 24 may be supported remotely from the surface 16 by the suture receiver support 22, and the tissue receiver support base 18 may be supported remote relative to the surface 16 by the suture receiver support 22. Thus, the suture receiver support 22 may hold the suture receiver 24, the tissue receiver support base 18 and the tissue receiver 19 above a support base 18.

The suture receiver 24, as shown in Figure 3-7, 9-22, may be configured to enable a stitch to be formed in the tissue 12, and the suture 14 to be looped around the tissue 12 while maintaining the suture 14 under constant tension by receiving a portion of a suture under tension 26 extending from a tissue 12 held by the tissue receiver 19, whereby the portion of the suture under tension 26 remains under tension by force applied to the suture 14 such that the portion of the suture under tension 26 is manipulated from a first side 28 of the tissue securement assembly 35 and suture receiver 24 to a second side 30 of the tissue securement assembly 35 and suture receiver 24. As such, a user may be better able to create whip stitching and the like during graft preparation by keeping the suture 14 under constant tension.

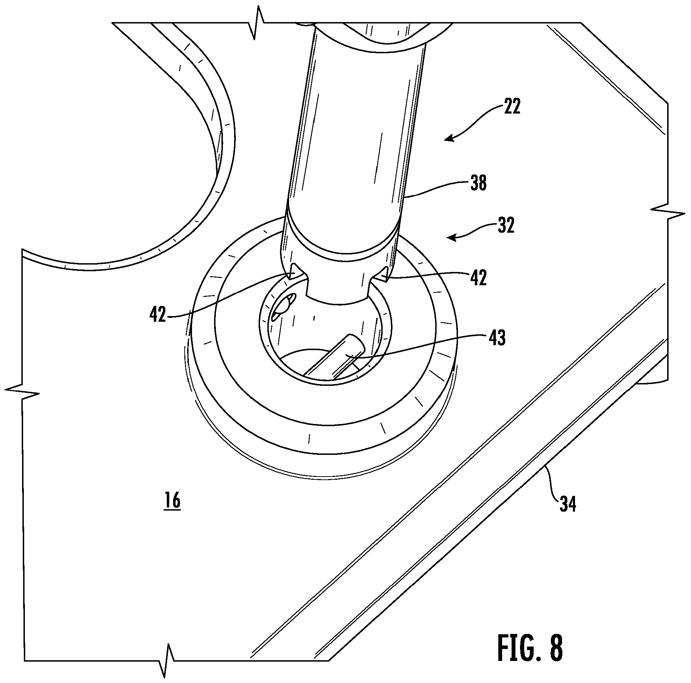

In at least one embodiment, the graft support system 10 may include a suture receiver support 22 configured to support the tissue receiver support base 18 and the suture receiver 24 remote from the surface 16. The suture receiver support 22 may be formed from one or more support members 38 configured to be removably attached to the surface 16. In at least one embodiment, the support member 38 may be, but is not limited to being, a shaft. The shaft may have a cylindrical cross-sectional shape or other shaped cross-section. The support member 38 may be configured to be removably attached to the surface 16. In at least one embodiment, the support member 38 may include one or more releasable connection systems 32 positioned distally of the suture receiver 24. In at least one embodiment, the releasable connection system 32 may be positioned on a distal end 40 of the support member 38 and may include one or more slots 42 configured to mate with protrusions 43, as shown in Figure 8, in a base structure configured to receive the support member 38. The slots 42 assist in preventing rotation of the support member 38 within the base structure.

The graft support system 10 may include a system base 34 configured to receive the support member 38 and to enable the support member 38 to extend from and to support a piece of tissue 12 in the tissue securement assembly 35 above the system base 34. The system base 34 may also include a second tissue securement assembly 36. The system base 34 may have any configuration enabling the first and second tissue securement assemblies 35, 36 to extend away from the system base 34 orthogonally, or nonorthogonally and nonparallel to the system base 34. The first and second tissue securement assemblies 35, 36 may be fixedly or movably attached to the system base 34. In at least one embodiment, the system base 34 may be configured to rest on top of a benchtop, tabletop or the like. In other embodiments, the system base 34 may be fixedly attached to a support structure such as, but not limited to a bench or a table. The suture receiver 24 may be supported above the system base 34 via the suture receiver support 22, such as, but not limited to, the one or more support members 38.

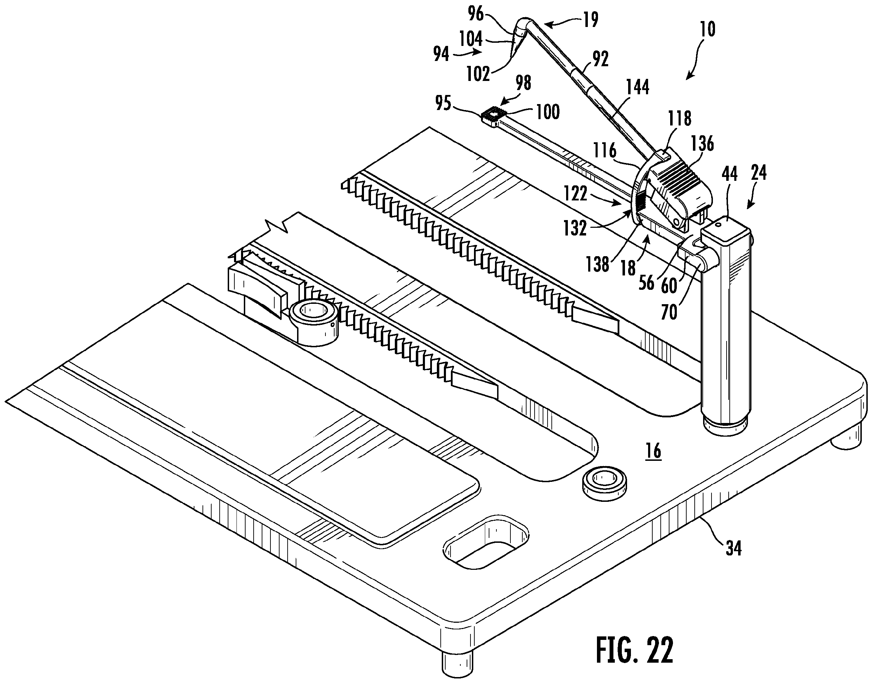

In another embodiment, as shown in Figure 22, the suture receiver 24 may be configured to support one or more tissue receivers 19, which may be first tissue securement assembly 35, above a surface 16, such as, but not limited to, a system base 34. In other words, the suture receiver 24 may be sized and configured to rest on or otherwise be attached to the surface 16 or other structure or surface and enable a tissue securement assembly 35 to hold a piece of tissue 12 sufficiently far enough above the surface 16 to enable a user to manipulate a suture 14 from one side to the other and to stitch the tissue 12. In such as configuration, the suture receiver 24 may include a suture receiver support 22 integrally formed within the suture receiver 24. In at least one embodiment, the suture receiver 24 may be attached to a tissue receiver support base 18 or otherwise secured in place, such as to a bench, tabletop or other surface or support.

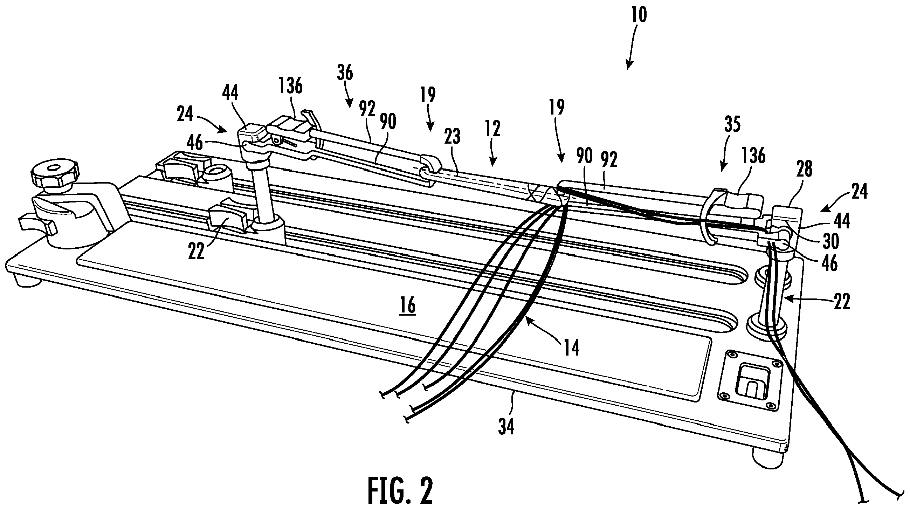

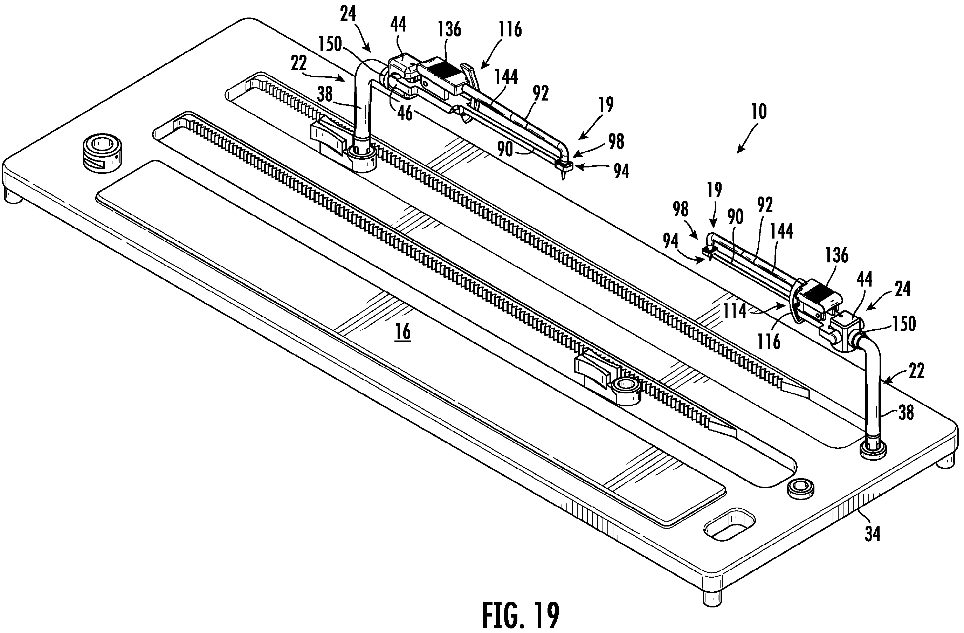

In at least one embodiment, as shown in Figure 19, the graft support system 10 may include a rotatable support structure 150 configured to enable the first and second tissue securement assemblies 35, 36 to be rotated along an axis generally aligned with a longitudinal axis of the tissue material 12 extending between the first and second tissue securement assemblies 35, 36. The rotatable support structure 150 may enable a user to easily work on and observe the tissue material 12. The description of the graft support system 10 herein describes the first tissue securement assembly 35 as including a suture receiver 24 enabling a suture 14 attached to the tissue 12 to remain under tension while being passed from one side 28 to another side 30 without having to pass the suture from one hand to another and possibly lose tension. In at least one embodiment, both the first and second tissue securement assemblies 35, 36 include suture receivers 24 and one or more of the other components set forth herein, as shown in Figures 2, 18 and 19.

The suture receiver 24 may enable a suture 14 attached to the tissue 12 to remain under tension while being passed from one side 28 to another side 30 without having to pass the suture from one hand to another and possibly lose tension. In particular, the suture receiver 24 may be configured to enable a stitch to be formed in the tissue, and the suture 14 to be looped around the tissue while maintaining the suture 14 under constant tension by receiving a portion of a suture under tension 26 extending from the tissue 12 held within the tissue receiver 19. The portion of the suture under tension 26 remains under tension by force applied to the suture 14 by a user such that the portion of the suture 14 under tension is manipulated from a first side 28 of the tissue securement assembly 35 and suture receiver 24 to a second side 30 of the tissue securement assembly 35 and suture receiver 24.

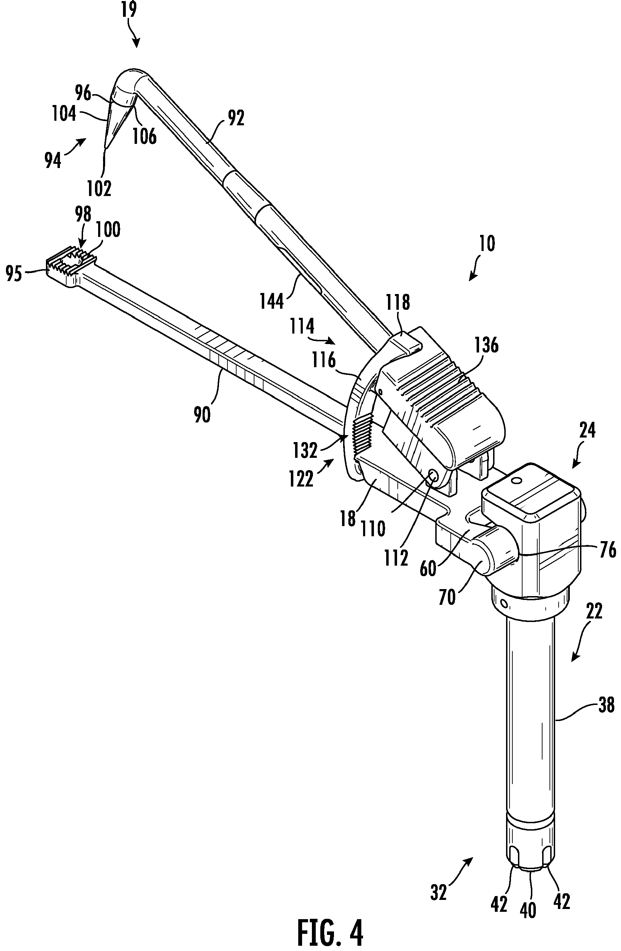

In at least one embodiment, the suture receiver 24 may include a suture receiver base 44 configured to slidably receive a suture receiver body 46 whereby, in a first position 48, as shown in Figures 3, 9, 12, 13, 15 and 17, a suture receiving opening 50 is open to receive a suture 14 under tension from the first side 28 of the tissue securement assembly 35 and suture receiver 24 and, in a second position 52, as shown in Figures 4, 11, 14 and 16, a suture release opening 54 is open to release a suture 14 under tension from the second side 30 of the tissue securement assembly 35 and suture receiver 24. The suture receiver body 46 may include a base support member 56 and first and second arms 58, 60 separated to form a suture receiving chamber 62. In such configuration, the suture receiver 24 may be configured to move the suture receiver base 44 between the first position 48, as shown in Figure 9, thru the middle position, as shown in Figure 10, to the second position 52, as shown in Figure 11.

In at least one embodiment, the suture receiver 24 may be formed from a suture receiver body 46 that is slidable relative to the suture receiver base 44. The suture receiver body 46 may include a base support member 56 and first and second arms 58, 60 separated to form a suture receiving chamber 62. The suture receiving chamber 62 may have any appropriate configuration enabling a suture to be received therein and moved from a first side 28 to a second side 30 of the tissue securement assembly 35 and suture receiver 24. In a first position 48, a suture receiving opening 50 is open on a first side 28 of the tissue securement assembly 35 and suture receiver 24. At the same time, the suture release opening 54 on the second side 30 is closed and the second arm 60, which is on the second side 30, is supporting the suture receiver 24. In the first position 48, the first side 28 of the suture receiver 24, in particular, the first arm 58, is unsupported. As such, a suture 14 is capable of being inserted into the suture receiving opening 50 on the first side 28 of the suture receiver 24 without obstruction. The suture receiving opening 50 receives a suture 14 under tension from the first side 28 of the suture receiver 24 via the suture receiving opening 50.

The suture receiver base 44 of the suture receiver 24 may be moved to a second position 52 in which the suture release opening 54 is open and the suture receiving opening 50 is closed. In this position, the suture receiver 24 is supported by the first side 28 of suture receiver 24 and releases a suture 14 under tension from the second side 30 of the suture receiver 24 via the suture release opening 54.

The suture receiver 24 may also include a retention mechanism 64 configured to releasably attach the suture receiver 24 to the suture receiver base 44. In at least one embodiment, the retention mechanism 64 may be, but is not limited to being a first retainer 66 on a distal end 68 of the first arm 58 and a second retainer 70 on a distal end 72 of the second arm 60. The first and second retainers 66, 70 may be aligned along a longitudinal axis 74 enabling the suture receiver body 46 to be attached to the suture receiver base 44 by sliding the first and second retainers 66, 70 into at least one receiver 76 in the suture receiver base 44. In at least one embodiment, the first and second retainers 66, 70 may be, but are not limited to being, cylindrical. The cross-sectional shape of the first and second retainers 66, 70 may be another appropriate configuration other than cylindrical still enabling the suture receiver body 46 to slide relative to the suture receiver base 44 when in use. The suture receiver base 44 may be configured to slidably retain the suture receiver body 46 such that the movement of the suture receiver body 46 is limited to movement along a single axis 73, which in at least one embodiment, is aligned, and, in at least one embodiment, is coaxial, with the longitudinal axis 74.

The graft support system 10 may also include a suture receiver body restraint 78 configured to limit movement of the suture receiver support 46 between the first and second positions 48, 52 while maintaining support of the tissue receiver support base 18 by the suture receiver support 22. In particular, the suture receiver body restraint 78 ensures that the suture receiver body 46 and tissue securement assembly 35 attached thereto is supported either by the first arm 58 or the second arm 60 and prevents the suture receiver body 46 and tissue securement assembly 35 from falling off the suture receiver base 44. In at least one embodiment, the suture receiver body restraint 78 may include one or more members extending from the suture receiver base 44 into the suture receiving chamber 62. The suture receiver body restraint 78 may be, but is not limited to being, a pin 80 positioned in the suture receiving chamber 62 and attached to the suture receiver base 44. As the suture receiver body 46 moves laterally, the suture receiver body 46 contacts the suture receiver body restraint 78 and movement stops.

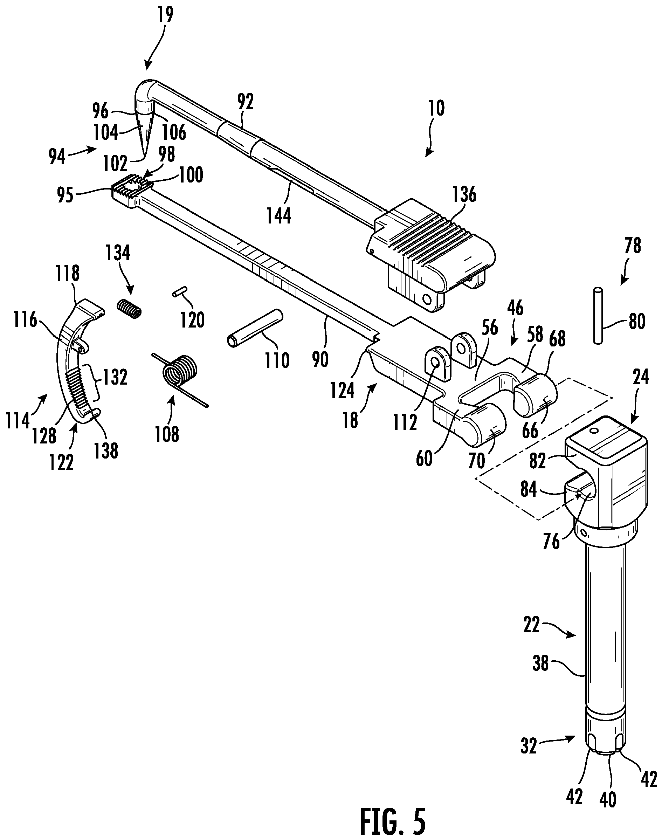

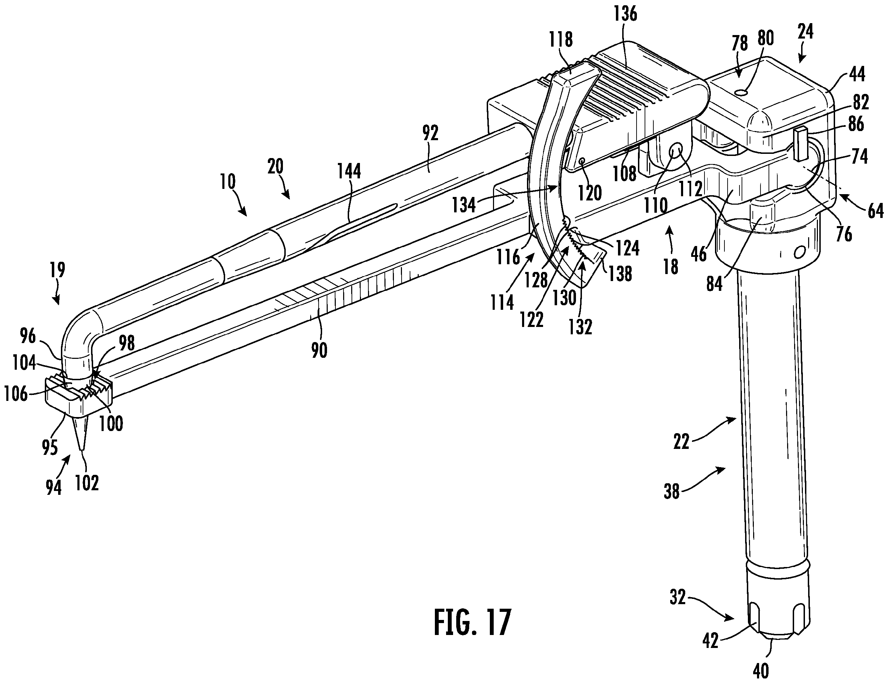

In at least one embodiment, the suture receiver body restraint 78 may be a pin 80, as shown in Figures 5, 6, 13, 15, 16 and 17 extending from a first portion 82 of the suture receiver base 44 to a second portion 84 of the suture receiver base 44 and positioned within the suture receiving chamber 62. In another embodiment, as shown in Figure 17, the suture receiver body restraint 78 may include one or more members 86 extending from the suture receiver body 46 to limit movement of the suture receiver body 46 between the first and second positions 48, 52 while maintaining support of the tissue receiver support base 18 by the suture receiver support 22. In at least one embodiment, the suture receiver body restraint 78 may include one or more members 86 extending from the suture receiver body 46 or one or more members 80 extending from the suture receiver base 44, or both, to limit movement of the suture receiver body 46 between the first and second positions 48, 52 while maintaining support of the tissue receiver support base 18 by the suture receiver support 22.

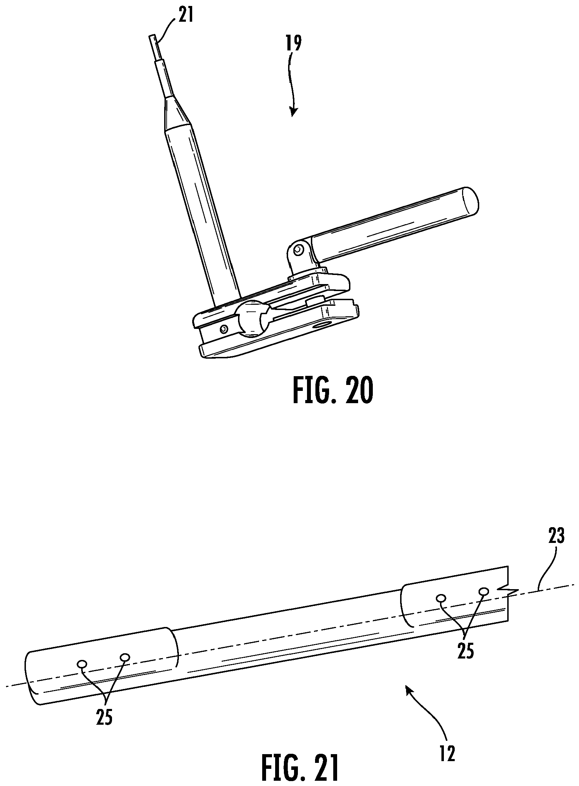

The graft support system 10 may also include one or more tissue receivers 19. In at least one embodiment, the tissue receiver 19 may be one or more posts 21, as shown in Figure 20, extending nonorthogonal and nonparallel to a longitudinal axis 23 of a graft 12 extending between first and second tissue receiver assemblies 35, 36. In at least one embodiment, one or more posts 21 may be positioned orthogonal to the longitudinal axis 23 of a graft 12 extending between first and second tissue receiver assemblies 35, 36. As such, the post 21 may receive a piece of material 12, such as a graft, such that the post 21 secures the piece of material 12 once the post 21 is inserted into a hole 25, as shown in Figure 21, in the piece of material 12, such as, but not limited to being, a graft. The post 21 enables a graft 12 to be easily attached to and detached from the graft support system 10.

The tissue receiver 19 is not limited to being a post 21 but may also be any other structure configured to retain a piece of material 12. In at least one embodiment, the tissue receiver 19 may be one or more tissue clamps 35 coupled to the tissue receiver support base 18 and configured to grasp a graft 12, as shown in Figures 2-7, 12, 14 and 17-19. The tissue clamp 35 may be formed from first and second clamp arms 90, 92, whereby the second arm 92 is pivotably coupled to the first arm 90 at the tissue receiver support base 18. The second clamp arm 92 may include a graft punch 94 at a distal end 96 of the second clamp arm 92, and the first clamp arm 90 may include a punch receiver 96. In at least one embodiment, the graft punch 94 may extend orthogonally from the second clamp arm 92, and in other embodiments, may extend nonorthogonally and nonparallel, from the second clamp arm 92.

In at least one embodiment, the punch receiver 98 may be an orifice 100 in the first clamp arm 90 near the distal end 95 of the first clamp arm 90 and aligned with the graft punch 94 on the second clamp arm 92 such that at least a portion of the graft punch 94 extends into the orifice 100 in the first clamp arm 90 when in a closed position. In a closed positioned of at least one embodiment, the distal end 102 of the graft punch 94 may extend into and through the orifice 100 in the first clamp arm 90. The orifice 100 may have any appropriate size. In at least one embodiment, the orifice 100 may be sized to be slightly smaller than an outer diameter 104 of a base 106 of the graft punch 94. As such, the orifice 100 stops movement of the graft punch 94 when the base 106 of the graft punch 94 contacts the first clamp arm 90 forming the orifice 100. The orifice 100 may stop movement of the second clamp arm 92 from rotating towards the first clamp arm 90 when the second clamp arm 92 is generally parallel with the first clamp arm 90.

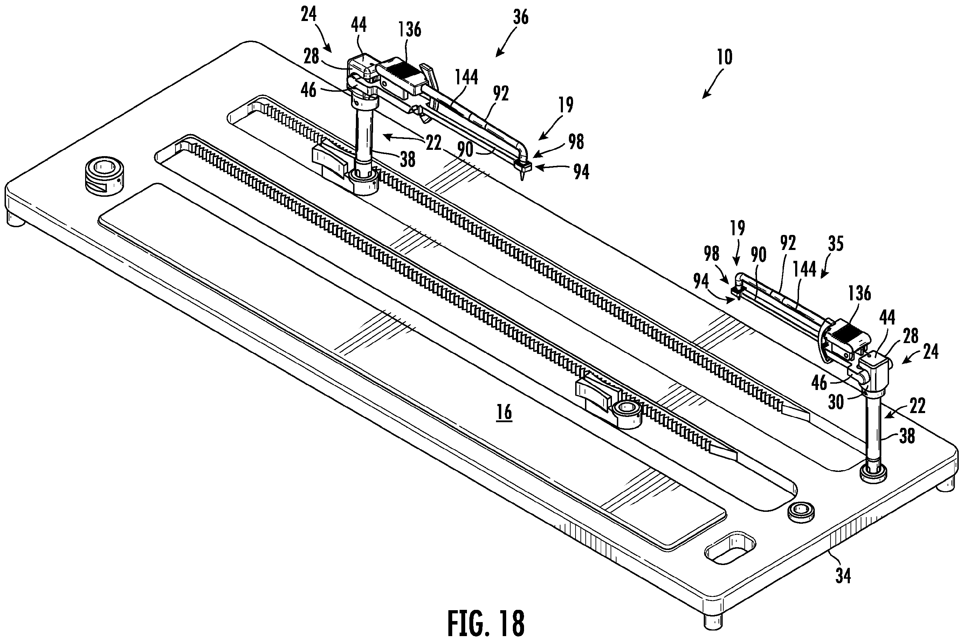

In other embodiments, the first and second clamp arms 90, 92 may be configured with distal ends having any configuration enabling the first and second clamp arms 90, 92 to grasp tissue. The first and second clamp arms 90, 92 may be configured identically, as shown in Figures 18 and 19, or differently, as shown in Figure 2. The first and second clamp arms 90, 92 may include grip enhancements, such as, but not limited to, teeth, multiple points, jagged edges, knurled surfaces, etched surfaces, abrasives and adhesives. In at least one embodiment, one or more of the first and second clamp arms 90, 92 may include convention clamp ends.

The second clamp arm 92 may be biased to rotate away from the first spring arm 90. In at least one embodiment, a spring 108, such as, but not limited to, a torsion spring, as shown in Figure 5, may be used to bias the second clamp arm 92 away from the first spring arm 90. The torsion spring may be positioned proximate to a pin 110 forming a pivot point 112 for the second clamp arm 92 relative to the first spring arm 90.

The graft support system 10 may also include a tissue receiver support base 18 configured to support one or more tissue clamps 35. The tissue receiver support base 18 may have any appropriate configuration capable of supporting a tissue clamp 35. In at least one embodiment, the tissue receiver support base 18 may be a generally planar member configured to enable a clamp 35 to be attached thereto. The first clamp arm 90 may extend from a distal end of the tissue receiver support base 18. The second clamp arm 90 may also be rotatably coupled to the tissue receiver support base 18. In at least one embodiment, as shown in Figures 3-8, 12, 14 and 17, the second clamp arm 90 may be rotatably coupled to the tissue receiver support base 18 between distal and proximal ends of the tissue receiver support base 18.

The graft support system 10 may also include a clamp arm retainer 114 configured to releasably secure the second clamp arm 92 in a fixed position relative to the first clamp arm 90. The clamp arm retainer 114 may include a retainer arm 116 pivotably coupled to the second clamp arm 92 at a pivot point 120. The retainer arm 116 may include an actuator end 118 configured to be actuated by a user by pivoting the actuator end 118 of the retainer arm 116 relative to the pivot point 120 where the retainer arm 116 is pivotably coupled to the second clamp arm 92.

The retainer arm 116 may include an engagement system 122 configured to releasably contact the first clamp arm 90 to retain the second clamp arm 92 in a desired position relative to the first clamp arm 90. As such, the graft support system 10 may be used to grab and hold a piece of tissue 12, such as, but not limited to, a graft, via the graft punch 94. In at least one embodiment, the engagement system 122 may include protrusion 124, such as a tooth 124 or other such protrusion, that may extend from a tissue receiver support base 18 and may be configured to be received in one or more grooves 128 on a surface 130 of the retainer arm 116 facing the tooth 124. In at least one embodiment, the one or more grooves 128 may be a plurality of grooves 128. The grooves 128 may extend into the surface 130 and may be formed with one more teeth extending from the surface and the like. Alternatively, a configuration resembling a gear rack 132 may be positioned on the surface 130 of the retainer arm 116 facing the tooth 124. The gear rack 132 may be attached to or may be formed integrally within the retainer arm 116.

The graft support system 10 may also include a retainer arm biasing system 134 configured to bias the retainer arm 116 into contact with a stop feature 138 in communication with the first clamp arm 90. The retainer arm biasing system 134 may be configured to bias the one or more grooves 128 on the surface 130 of the retainer arm 116 facing the tooth 124 towards the tooth 124 on the first clamp arm 90. As such, the second clamp arm 92 is prevented from moving unless a user pulls the actuator end 118 of the retainer arm 116 against the biasing force, which in at least one embodiment is towards a thumb pad 135 on the second clamp arm 92. In such configuration, the second clamp arm 92 remains temporarily fixed in position at all times with the retainer arm 116 biased against the tooth 124 until a user disengages the retainer arm 116 by pressing the actuator end 118 towards the thumb pad 136.

The graft support system 10 may also include a stop 138 configured to limit movement of the second clamp arm 92 relative to the first clamp arm 90 so the second clamp arm 92 doesn't open too wide. In at least one embodiment, the stop 138 may extend from the retainer arm 116 towards the first clamp arm 90 such that the stop 138 may contact the first clamp arm 90, the tissue receiver support base 18 or another component when the second clamp arm 92 is rotated away from the first clamp arm 90, thereby limiting movement of second clamp arm 92. The stop 138 may be any member of sufficient size and strength to limit movement of the second clamp arm 92, such as, but not limited to, by contacting the first clamp arm 90 or the tissue receiver support base 18.

The graft support system 10 may also include a suture card receiver slot 144 in the second clamp arm 92, as shown in Figures 3-7, 12, 14 and 17, which enables an implant card 146, as shown in Figure 1, to be held in place on the second clamp arm 92 thereby enabling sutures 14 to be held in place on the implant card 146 while one or more sutures 14 extending from the implant card 146 is attached to a piece of tissue material 12, such as, but not limited to being, a graft, held by the graft support system 10. The graft support system may comprise a suture card receiver slot in the second clamp arm enabling the second clamp arm to receive an implant card containing a suture which enables sutures to be stored in close proximity to the site of use in the tissue.

During use, the graft support system 10 may be used to grasp and hold a piece of tissue 12 under tension above a surface 16, which in at least one embodiment as shown in Figures 2, 7, 18, 19 and 22, entails the first and second tissue securement assemblies securely grasping a piece of tissue 12 to support the tissue 12 above the support base 34 such that suture attached to the tissue 12 to remains under tension while being passed from one side 28 to another side 30 without having to pass the suture 14 from one hand to another and possibly lose tension. A piece of tissue 12 may be held securely by the second tissue securement assembly 36. The piece of tissue 12 may be pulled tight and moved between the distal ends 95, 96 of the first and second clamp arms 90, 92 so that the graft punch 94 may grasp the piece of tissue 12 and retain the tissue 12 extending between the first and second tissue securement assemblies 35, 36. With the first and second clamp arms 90, 92 in an open position in which the graft punch 94 is separated from the orifice 100, such as when the first and second clamp arms 90, 92 are separated to a maximum open position as limited by stop 138, the piece of tissue 12 may be properly positioned for engagement and the second clamp arm 92 may be pushed towards the first clamp arm 90 via the thumb pad 136 or by pushing the second clamp arm 96 towards the first clamp arm 90.

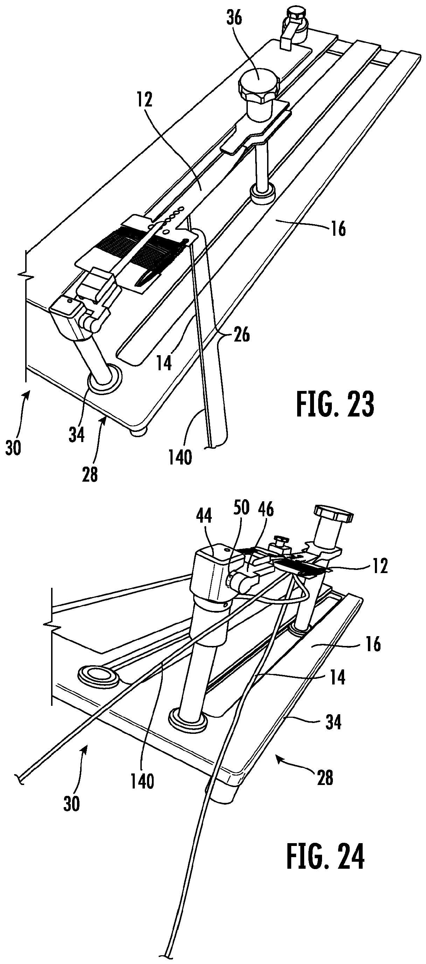

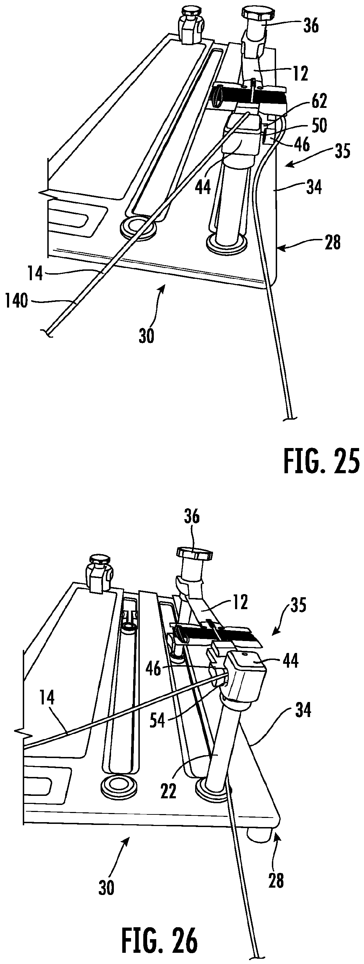

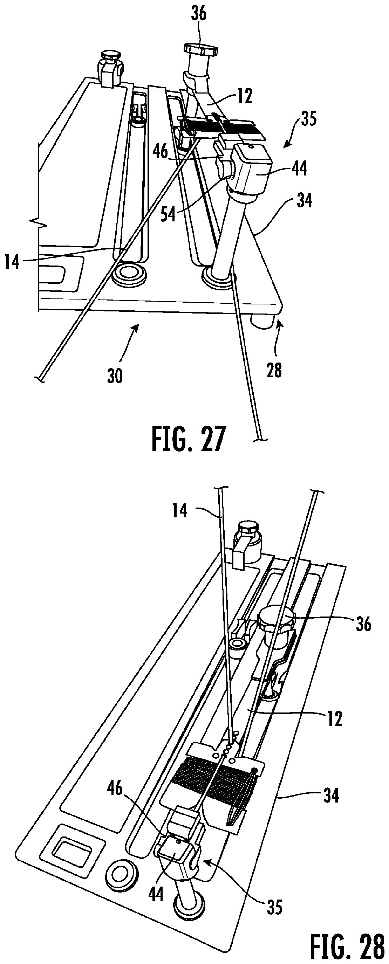

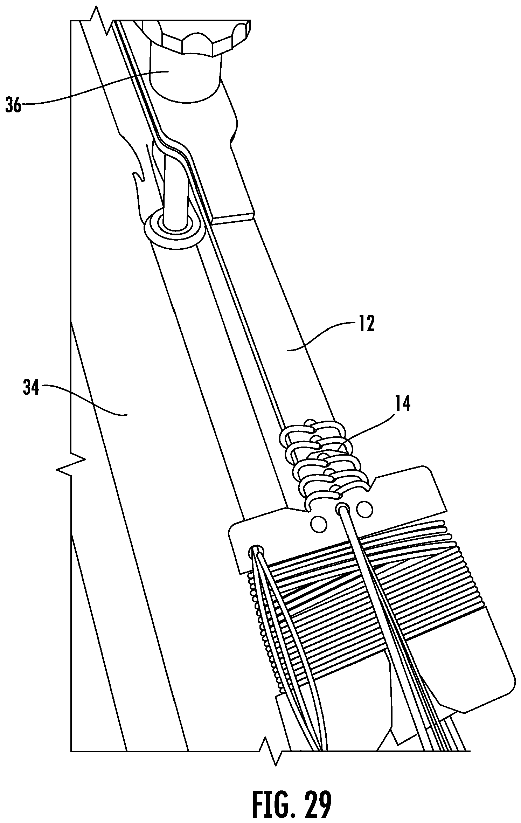

Once the piece of tissue 12 is secured and extending between the first and second tissue securement assemblies 35, 36, a needle may be passed through the tissue 12 and the suture 14 pulled tight to place the suture 14 under tension, as shown in Figure 24. The tag end 140 of the suture 14 may be pulled tight to place the suture under tension. The portion of the suture under tension 26 may be pulled into the suture receiving chamber 62, as shown in Figure 25, via the suture receiving opening 50 of the suture receiver 24 on the first side 28 of the tissue securement assembly 36 and suture receiver 24. In such process, the portion of the suture under tension 26 may remain under constant tension. The suture receiver body 46 may then be moved from the first position 48 to the second position 52 whereby the suture release opening 54 is opened and the suture receiving opening 50 is closed. The suture under tension 26 may be moved within the suture receiving chamber 62, as shown in Figure 25 and pulled out of the suture release opening 54, as shown in Figures 26 and 27. Doing so enables the suture 14 under tension to be moved from the first side 28 of the tissue securement assembly 35 and suture receiver 24 to the second side 30. In other words, a user may pull the suture 12 tight and move the suture from the first side 28 of the tissue securement assembly 35 and suture receiver 24 to the second side 30 of the tissue securement assembly 35 and suture receiver 24 while keeping the suture under tension. The tensioned tag end 140 of the suture 14 is then ready for the next stitch, as shown in Figure 28. The fully prepare piece of tissue 12 is shown in Figure 29.

A method of preparing a graft for implantation or other use may include graft support system 10, e.g. a graft support system as disclosed herein, being used to grasp and hold a piece of tissue 12 under tension above a surface 16, which in at least one embodiment as shown in Figure 23, and may entail the first and second tissue securement assemblies securely grasping a piece of tissue 12 to support the tissue 12 above the support base 34 such that suture attached to the tissue 12 remains under tension while being passed from one side 28 to another side 30 without having to pass the suture 14 from one hand to another and possibly lose tension. The method may include a piece of tissue 12 being held securely by the second tissue securement assembly 36. The piece of tissue 12 may be pulled tight and moved between the distal ends 95, 96 of the first and second clamp arms 90, 92 so that the graft punch 94 may grasp the piece of tissue 12 and retain the tissue 12 extending between the first and second tissue securement assemblies 35, 36. With the first and second clamp arms 90, 92 in an open position in which the graft punch 94 is separated from the orifice 100, such as when the first and second clamp arms 90, 92 are separated to a maximum open position as limited by stop 138, the piece of tissue 12 may be properly positioned for engagement and the second clamp arm 92 may be pushed towards the first clamp arm 90 via the thumb pad 136 or by pushing the second clamp arm 96 towards the first clamp arm 90.

The method of preparing a graft may include, once the piece of tissue 12 is secured and extending between the first and second tissue securement assemblies 35, 36, passing a needle through the tissue 12 and pulling the suture 14 tight to place the suture 14 under tension, as shown in Figure 24. The method may include pulling the tag end 140 of the suture tight to place the suture under tension. The portion of the suture under tension 26 may be pulled into the suture receiving chamber 62, as shown in Figure 25, via the suture receiving opening 50 of the suture receiver 24 on the first side 28 of the tissue securement assembly 35 and suture receiver 24. In such method, the portion of the suture under tension 26 may remain under constant tension. The method may include moving the suture receiver body 46 from the first position 48 to the second position 52 whereby the suture release opening 54 is opened and the suture receiving opening 50 is closed. The method may include moving the suture under tension 26 within the suture receiving chamber 62, as shown in Figure 25, and out of the suture release opening 54, as shown in Figures 26 and 27. Doing so enables the suture 14 under tension to be moved from the first side 28 of the tissue securement assembly 35 and suture receiver 24 to the second side 30 of the tissue securement assembly 35 and suture receiver 24. In other words, a user may pull the suture 12 tight and move the suture from the first side 28 of the tissue securement assembly 35 and suture receiver 24 to the second side 30 while keeping the suture under tension. The tensioned tag end 140 of the suture 14 is then ready for the next stitch, as shown in Figure 28. The method may be replicated numerous times until a piece of tissue has been fully prepared, as shown in Figure 29.

Typical graft tissue preparation required a user to pass the suture from one side to the other side of the graft underneath the graft while trying to maintain tension on the graft. Such was difficult and wasted time. In addition, it was not always possible to maintain tension on the suture. The graft support system 10 and the method of using the graft support system 10 enables a user, such as, but not limited to, a medical professional, to keep tension on a suture in preparation for another stitch when moving a tensioned tag end of the suture from one side of a clamp to a second side of the clamp without having to pass the suture from one hand to another. Rather, the tensioned tag end of the suture can remain in the same hand of the user. Such system and method saves time and greatly increases the likelihood that tension on the suture continue uninterrupted while the user is stitching the graft in preparation for implantation.

The foregoing is provided for purposes of illustrating, explaining, and describing embodiments of this invention. Modifications and adaptations to these embodiments will be apparent to those skilled in the art and may be made without departing from the scope or spirit of this invention. Upon reviewing the aforementioned embodiments, it would be evident to an artisan with ordinary skill in the art that said embodiments can be modified, reduced, or enhanced without departing from the scope and spirit of the claims described below.

## Claims

1. A graft support system (10), comprising:
at least one tissue receiver (19) configured to retain a tissue (12);
a suture receiver (24) supported remotely from at least one surface (16) and configured to support the at least one tissue receiver (19) remote relative to the at least one surface (16) such that material (12) can be passed between the at least one surface (16) and the tissue (12) supported by the graft support system (10); and
wherein the suture receiver (24) is configured to enable a stitch to be formed in the tissue (12) and a suture (14) to be looped around the tissue (12) while maintaining the suture (14) under constant tension by receiving a portion of a suture under tension (26) extending from the tissue (12) held within the at least one tissue receiver (19), whereby the portion of the suture under tension (26) remains under tension by force applied to the suture (14) when the portion of the suture under tension (26) is manipulated from a first side (28) of the suture receiver (24) to a second side (30) of the suture receiver (24).

2. The graft support system (10) of claim 1, wherein the suture receiver (24) comprises a suture receiver base (44) slidably receiving a suture receiver body (46) whereby, in a first position, a suture receiving opening (50) is open to receive a suture under tension (26) from the first side (28) of the suture receiver (24) and, in a second position, a suture release opening (54) is open to release a suture under tension (26) from a second side (30) of the suture receiver (24).

3. The graft support system (10) of claim 2, wherein the suture receiver body (46) comprises a base support member and first and second arms separated to form a suture receiving chamber, and wherein
the graft support system (10) may further comprise a first retainer on a distal end of the first arm and a second retainer on a distal end of the second arm.

4. The graft support system (10) of claim 2, wherein the suture receiver base (44) slidably retains the suture receiver body (46) such that the movement of the suture receiver body (46) is limited to movement along a single axis, or wherein
the graft support system (10) further comprises a suture receiver body restraint (78) configured to limit movement of the suture receiver body (46) between the first and second positions, wherein the suture receiver body restraint (78) comprises at least one member extending from the suture receiver body (46) to limit movement of the suture receiver body (46) between the first and second positions along an axis while maintaining support of a tissue receiver support base (18) by the suture receiver body (46).

5. The graft support system (10) of claim 1, wherein the at least one tissue receiver (19) comprises at least one post configured to receive the tissue (12).

6. The graft support system (10) of claim 1, wherein the at least one tissue receiver (19) comprises at least one tissue clamp (35).

7. The graft support system (10) of claim 6, wherein the at least one tissue clamp (35) extends from a tissue receiver support base (18), which is in turn supported by the suture receiver (24), and wherein the at least one tissue clamp (35) is configured to grasp the tissue (12).

8. The graft support system (10) of claim 7, wherein the at least one tissue clamp (35) is formed from first (90) and second (92) clamp arms, whereby the second clamp arm (92) is pivotably coupled to the first clamp arm (90) at the tissue receiver support base (18), and wherein
the graft support system (10) may further comprise a clamp arm retainer (114) configured to releasably secure the second clamp arm (92) in a fixed position relative to the first clamp arm (90), and wherein
the graft support system (10) may further comprise a suture card receiver slot (144) in the second clamp arm (92) enabling the second clamp arm (92) to receive an implant card containing a suture (14) which enables sutures to be stored in close proximity to the site of use in the tissue (12).

9. The graft support system (10) of claim 1, further comprising a suture receiver support (22) configured to support the suture receiver (24) remote relative to the at least one surface (16).

10. The graft support system (10) of claim 9, further comprising at least one releasable connection system (32) configured to enable the suture receiver support (22) to be releasably attachable to the at least one surface (16).

11. The graft support system (10) of claim 1, further comprising a rotatable support structure (150) configured to enable the at least one tissue receiver (19) to be rotated along an axis generally aligned with the tissue (12) supported by the graft support system (10).

12. A graft support system (10), comprising:
at least one tissue receiver (19) configured to retain a tissue (12);
a suture receiver (24) supported remotely from at least one surface (16) and configured to support the at least one tissue receiver (19) remote relative to the at least one surface (16) such that material (12) can be passed between the at least one surface (16) and the tissue supported by the graft support system (10);
wherein the suture receiver (24) is configured to enable a stitch to be formed in the tissue (12) and a suture to be looped around the tissue (12) while maintaining the suture under constant tension by receiving a portion of a suture under tension (26) extending from the tissue (12) held within the at least one tissue receiver (19), whereby the portion of the suture under tension (26) remains under tension by force applied to the suture when the portion of the suture under tension (26) is manipulated from a first side (28) of the suture receiver (24) to a second side (30) of the suture receiver;
wherein the suture receiver (24) comprises a suture receiver base (44) slidably receiving a suture receiver body (46) whereby, in a first position, a suture receiving opening (50) is open to receive a suture under tension (26) from the first side (28) of the suture receiver (24) and, in a second position, a suture release opening (54) is open to release a suture under tension (26) from a second side (30) of the suture receiver (24); and
wherein the at least one tissue receiver (19) comprises at least one tissue clamp (35).

13. A method of preparing a graft, comprising:
supporting a piece of tissue (12) via a graft support system (10) such that the piece of tissue (12) is supported away from at least one surface (16);
placing at least a portion of a tag end (140) of a suture under tension (26) such that the suture attached to the piece of tissue (12) is under tension; and
passing the tag end (140) of the suture (14) from a first side (28) of the graft support system (10) to a second side (30) of the graft support system (10) while retaining the suture and at least a portion of a tag end under tension such that the tag end (140) of the suture (14) is in position to create another stitch within the piece of tissue (12).

14. The method of preparing a graft of claim 13, wherein passing the tag end (140) of the suture (14) from a first side (28) of the graft support system (10) to a second side (30) of the graft support system (10) while retaining the suture and at least a portion of a tag end under tension comprises passing the tag end (140) of the suture from a first side (28) of the graft support system (10) to a second side (30) of the graft support system (10) without a user having to relinquish grip of the tag end.

15. The method of preparing a graft of claim 14, wherein supporting a piece of tissue (12) via a graft support system (10) such that the piece of tissue (12) is supported away from a surface (16) comprises supporting a piece of tissue (12) via a graft support system (10) comprising a suture receiver (24) supported remotely from the at least one surface (16) and configured to support at least one tissue receiver (19) remote relative to the at least one surface (16) such that material (12) can be passed between the at least one surface (16) and the piece of tissue (12) supported by the graft support system (10), and wherein
the method of preparing a graft may further comprise:
wherein passing the tag end (140) of the suture (14) from a first side (28) of the graft support system (10) to a second side (30) of the graft support system (10) while retaining the suture and at least a portion of a tag end (140) of the suture under tension (26) comprises moving a portion of a tag end (140) of the suture under tension (26) on the first side (28) of the graft support system (10) into a suture receiving chamber of a suture receiver body (46) of the suture receiver, moving a suture receiver body (46) from a first position to a second position whereby a suture release opening (54) is opened and a suture receiving opening (50) is closed, and removing the tag end under tension from the suture release opening (54), thereby placing the tag end under tension on the second side (30) of the graft support system (10).
